# EUROPEAN PATENT SPECIFICATION

(11) **EP 2 945 599 B1**
(45) Date of publication and mention of the grant of the patent: **30.05.2018**
(21) Application number: 14701946.7
(22) Date of filing: 20.01.2014
(51) Int. Cl.: A61K 8/34, A61Q 5/06, A61K 8/73, A61K 8/81, A61K 8/04

(54) **COSMETIC COMPOSITION COMPRISING A SPECIFIC POLYSACCHARIDE, AN ANIONIC FIXING POLYMER AND A LIQUID MONOALCOHOL OR POLYOL, AND COSMETIC TREATMENT METHOD EMPLOYING IT**
KOSMETISCHE ZUSAMMENSETZUNG ENTHALTEND EIN SPEZIFISCHES POLYSACCHARID, EIN ANIONISCHES FIXIERENDES POLYMER UND EINEN FLÜSSIGEN MONOALKOHOL ODER POLYOL, UND KOSMETISCHES ANWENDUNGSVERFAHREN
COMPOSITION COSMÉTIQUE COMPRENANT UN POLYSACCHARIDE SPÉCIFIQUE, UN POLYMÈRE FIXANT ANIONIQUE ET UN MONOALCOOL OU POLYOL LIQUIDE, ET MÉTHODE DE COIFFAGE LA METTANT EN OEUVRE

(30) Priority: 18.01.2013 FR 1350455; 06.03.2013 US 201361773201 P
(43) Date of publication of application: 25.11.2015
(73) Proprietor: L'Oréal, 75008 Paris (FR)
(72) Inventor: FAVREAU, Valérie, F-95540 Méry-sur-Oise (FR)
(74) Representative: Leray, Noelle
(86) International application number: PCT/EP2014/051044
(87) International publication number: WO 2014/111578

(56) References cited:
- EP-A1- 1 719 545
- EP-A1- 1 767 183

## Description

The present invention relates to a cosmetic composition for the treatment of keratinous fibres, in particular human keratinous fibres, such as the hair, comprising one or more specific polysaccharide(s), one or more anionic fixing polymer(s) and one or more liquid monoalcohols or polyols, and also to a cosmetic treatment method employing it.

In the field of hair styling, in particular among hair products intended for the shaping and/or form retention of the hair style, hair compositions are generally provided in the form of gels, lotions, foams or hair sprays. The documents FR 2 729 563, EP 754 026, EP 1 532 966 and EP 1 629 827 describe, for example, hair styling compositions.

They are generally applied to wet hair, in particular after it has been washed and superficially dried, after a shower and superficial drying, for example. The use of these compositions requires an application time additional to the time spent under the shower.

Moreover, styling compositions generally leave, after hair styling, a tactile feeling of residual products in the hands, in particular a phenomenon of residual tackiness which remains on the hands, which requires that the hands be washed after the application of these compositions. A residual tackiness can also remain on the hair.

There thus exists a real need to find cosmetic compositions, in particular for hair styling, which make possible rapid styling and a good level of fixing and form retention of the hair style, while retaining a clean and non-tacky feel of the hair, which compositions can optionally be used directly in the shower, even without superficially drying.

A subject-matter of the present invention is thus in particular a cosmetic composition, in particular a hair cosmetic composition, comprising one or more polysaccharide(s) having at least one mannose unit, one or more anionic fixing polymer(s) and one or more liquid monoalcohols or polyols, the content of liquid monoalcohols or polyols being greater than or equal to 20% by weight of the total weight of the composition, the pH of the composition, when it is aqueous, being less than or equal to 6.5.

The combination according to the invention makes it possible to obtain a hair styling composition having a good level of fixing and rapid and easy hair styling. A hair styling composition is obtained which is rinsed out and which makes it possible to obtain a clean and non-tacky feel of the hair.

The composition according to the invention can in particular be used directly in the shower.

According to another of its aspects, the present invention relates to a method for the treatment of keratinous fibres, in particular for shaping the hair, in which the composition which has just been described is applied to the keratinous fibres and rinsed out afterwards. The expression "at least one" is equivalent to the expression "one or more".

The expression "hair composition" is understood to mean a composition which is applied to the hair, that is to say in particular for the form retention and/or fixing of the hair style, the care of the hair, the making up of the hair or the colouring of the hair.

The expression "styling composition" is understood to mean a composition which makes it possible to confer a shape on a head of hair and/or to retain the acquired shape of the head of hair.

### Polysaccharide

As indicated above, the cosmetic composition of the invention comprises at least one polysaccharide comprising at least one mannose unit.

Within the meaning of the present invention, the term "polysaccharide" is understood to denote any carbohydrate macromolecule formed by the linking of several elementary sugars, such as, for example, xylose, glucose, galactose, rhamnose, mannose, fucose, arabinose and their respective acids.

The polysaccharide according to the invention, comprising at least one mannose unit, is chosen from xanthans, galactomannans, glucomannans and their derivatives.

### Xanthan

Xanthan is a heteropolysaccharide produced on the industrial scale by the aerobic fermentation of the bacterium *Xanthomonas campestris.* Its structure is composed of a main chain of β-D-glucoses connected in β(1,4) fashion, similar to cellulose. One glucose molecule out of two carries a trisaccharide side chain composed of an α-D-mannose, of a β-D-glucuronic acid and of a terminal β-D-mannose. The internal mannose residue is generally acetylated on the 6 carbon. Approximately 30% of the terminal mannose residues carry a pyruvate group connected in the chelated form between the 4 and 6 carbons. The glucuronic acids and the charged pyruvic acids are ionizable and thus responsible for the anionic nature of xanthan (negative charge down to pH 1). The content of the pyruvate and acetate residues varies according to the bacterial strain, the fermentation process, the post-fermentation conditions and the purification stages. These groups can be neutralized in the commercial products with Na⁺, K⁺ or Ca²⁺ ions (Satia, 1986). The neutralized form can be converted into the acid form by ion exchange or by dialysis with an acid solution.

Chemical structure of the base unit of xanthan:

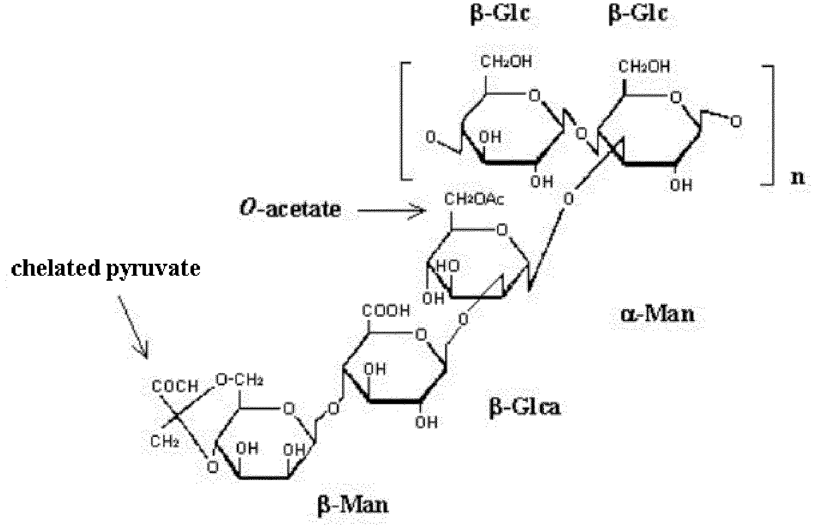

*Source: Christensen et al. (1993).*

Xanthan gums have a molecular weight of between 1 000 000 and 50 000 000 and a viscosity of between 0.6 and 1.65 Pa.s for an aqueous composition comprising 1% of xanthan gum (measured at 25°C using a Brookfield viscometer, LVT type, at 60 revolutions per minute).

Xanthan gums are represented, for example, by the products sold under the Rhodicare names by Rhodia Chimie, under the Satiaxane™ name by Cargill Texturizing Solutions (for the food, cosmetic and pharmaceutical industry), under the Novaxan™ name by ADM and under the Kelzan® and Keltrol® names by CP-Kelco.

**Galactomannans** (guar, locust bean, fenugreek, tara gum) and derivatives (phosphated guar, hydroxypropyl guar, and the like) :
Galactomannans are non-ionic polysaccharides extracted from the albumin of seeds of leguminous plants, of which they constitute the storage carbohydrate.

Galactomannans are macromolecules consisting of a main chain of D-mannopyranose units connected in β(1,4) fashion, carrying side branches consisting of a single D-galactopyranose unit connected in α(1,6) fashion to the main chain. The various galactomannans differ, on the one hand, in the proportion of α-D-galactopyranose units present in the polymer and, on the other hand, in significant differences in terms of distribution of the galactose units along the mannose chain.

The mannose/galactose (M/G) ratio is of the order of 2 for guar gum, of 3 for tara gum and of 4 for locust bean gum. The guar molecule thus has, on average, twice as many branches as the locust bean molecule. However, within one and the same sample, this ratio can vary depending on the fractions. The knowledge of the M/G ratio constitutes one of the means for characterizing the sample although it gives no information on the statistical distribution of the galactosyl residues on the main chain.

The galactomannans exhibit the following chemical structure:

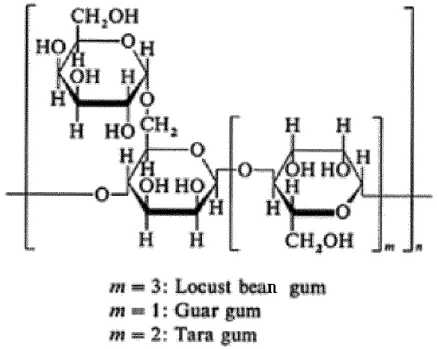

Tara gum is characterized by a more statistically random distribution of the side chains, while locust bean is characterized by a non-statistically random and non-uniform distribution, with an alternation of unsubstituted blocks of intermediate length (smooth regions) and branched blocks (hairy regions). Guar meal has very few smooth regions, with a very uniform distribution of the galactose side chains.

The galactomannans which can be used according to the invention comprise primary and secondary hydroxyl groups which, in principle, can be derived and substituted. At the industrial level, only the hydroxyalkyl, carboxymethyl and cationic derivatives, and also partially depolymerized grades, are marketed.

The hydroxyalkyl derivatives which can be used according to the invention are obtained, for example, by treating the galactomannans with ethylene oxide or propylene oxide in an alkaline medium. These derivatives differ from the starting material in their faster kinetics of hydration and their better solubility.

The products which can be used according to the invention can also be carboxymethylated derivatives. These derivatives are obtained by "etherification" of a certain portion of the hydroxyl groups with chloroacetic acid or sodium chloroacetate. Unlike carboxymethylcelluloses, which are soluble only from degrees of substitution of 0.7 to 1.0, galactomannans (if not already soluble before chemical modification) become completely soluble at a degree of substitution of less than 0.05.

The products which can be used according to the invention can also be cationic derivatives. Cationic derivatives are obtained by the treatment of the galactomannans with appropriate organic amines, such as 2-hydroxy-3-chloropropyltrimethylammonium chloride or its reaction product 2,3-epoxypropyltrimethylammonium chloride, which is formed in alkaline medium.

Finally, the products which can be used according to the invention can be partially depolymerized galactomannans, for this reason exhibiting reduced viscosities. These depolymerizations are obtained by acid hydrolysis, oxidation or enzymatic decomposition.

### Guar

Guar gum is characterized by a mannose:galactose ratio of the order of 2:1. The galactose group is uniformly distributed along the mannose chain.

The guar gums which can be used according to the invention can be non-ionic, cationic or anionic.

According to the invention, use may be made of unmodified or chemically modified non-ionic guar gums.

The unmodified non-ionic guar gums are, for example, the products sold under the names Vidogum GH, Vidogum G and Vidocrem by Unipektin and under the name Jaguar by Rhodia, under the name Meypro®Guar by Danisco, under the name Viscogum™ by Cargill and under the name Supercol® Guar Gum by Aqualon.

The hydrolysed non-ionic guar gums which can be used according to the invention are, for example, represented by the products sold under the Meyprodor® name by Danisco.

The modified non-ionic guar gums which can be used according to the invention are preferably modified by C₁-C₆ hydroxyalkyl groups.

Mention may be made, among the hydroxyalkyl groups, by way of example, of the hydroxymethyl, hydroxyethyl, hydroxypropyl and hydroxybutyl groups.

These guar gums are well known in the state of the art and can, for example, be prepared by reacting the corresponding alkene oxides, such as, for example, propylene oxides, with the guar gum, so as to obtain a guar gum modified by hydroxypropyl groups.

The degree of hydroxyalkylation, which corresponds to the number of alkylene oxide molecules consumed per the number of free hydroxyl functional groups present on the guar gum, preferably varies from 0.4 to 1.2.

Such non-ionic guar gums optionally modified by hydroxyalkyl groups are sold, for example, under the trade names Jaguar HP 60, Jaguar HP 105 and Jaguar HP 120 (hydroxypropyl guar) by Rhodia or under the name N-Hance® HP (hydroxypropyl guar) by Aqualon.

The cationic galactomannan gums preferably have a cationic charge density of less than or equal to 1.5 meq/g and more particularly of between 0.1 and 1 meq/g. The charge density can be determined according to the Kjeldahl method. It generally corresponds to a pH of the order of 3 to 9.

Generally, within the meaning of the present invention, the term "cationic galactomannan gum" is understood to mean any galactomannan gum comprising cationic groups and/or groups which can be ionized to give cationic groups.

The preferred cationic groups are chosen from those comprising primary, secondary, tertiary and/or quaternary amine groups.

The cationic galactomannan gums used generally have a weight-average molecular weight of between 500 and 5 x 10⁶ approximately and preferably of between 10³ and 3 x 10⁶ approximately.

The cationic galactomannan gums which can be used according to the present invention are, for example, gums comprising tri(C₁-C₄)alkylammonium cationic groups. Preferably, 2% to 30% by number of the hydroxyl functional groups of these gums carry trialkylammonium cationic groups.

Mention may very particularly be made, among these trialkylammonium groups, of the trimethylammonium and triethylammonium groups.

Example of cationic guar structure:

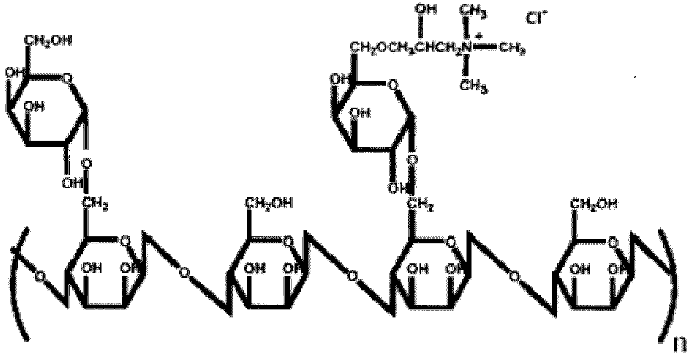

These galactomannan gums, in particular guar gums modified by cationic groups, are products already known per se and are, for example, described in the patents US 3 589 578 and US 4 031 307. Such products are furthermore sold in particular under the trade names of Jaguar Excel, Jaguar C13 S, Jaguar C 15, Jaguar C 17 and Jaguar C162 (Guar Hydroxypropyltrimonium Chloride) by Rhodia, under the name Amilan® Guar (Guar Hydroxypropyltrimonium Chloride) by Degussa and under the name N-Hance® 3000 (Guar Hydroxypropyltrimonium Chloride) by Aqualon.

The anionic guar gums which can be used according to the invention are polymers comprising groups derived from carboxylic acid, sulfonic acid, sulfenic acid, phosphoric acid, phosphonic acid or pyruvic acid. Preferably, the anionic group is a carboxylic acid group. The anionic group can also be provided in the form of an acid salt, in particular a sodium, calcium, lithium or potassium salt.

### Locust bean

Locust bean gum is extracted from carob tree (*Ceratonia siliqua*) seeds, which tree is an evergreen tree native to Syria and Asia Minor but now cultivated over the entire Mediterranean littoral.

The galactose sugars are not equally uniformly distributed along the chain formed of mannose units but have a tendency to be grouped together in blocks. The chains have a non-uniform structure with "smooth" regions and substituted regions alternating.

The locust bean gum which can be used in this invention can be chemically modified, according to the same chemical modifications as those described above for guar gum.

The non-modified locust bean gum which can be used in this invention is sold, for example, under the Viscogum™ name by Cargill, under the name Vidogum L by Unipektin or under the name Grinsted® LBG by Danisco.

The chemically modified locust bean gums which can be used in this invention can be represented, for example, by the cationic locust beans sold under the name Catinal CLB (Locust Bean Hydroxypropyltrimonium Chloride) by Toho and the locust beans modified by hydroxyethyl and carboxymethyl groups.

### Tara gum

Tara gum is extracted from the seed of *Caesalpinia spinosa* (Fam. Leguminosae), a South American tree.

The tara gum which can be used in this invention can be chemically modified, according to the same chemical modifications as those described above for guar gum.

The tara gum which can be used in the context of this invention is sold, for example, under the name Vidogum SP by Unipektin.

### Glucomannans

Glucomannans are polysaccharides, generally of high molecular weight (500 000 < Mglucomannan < 2 000 000), composed of D-mannose and D-glucose units. Generally, branching is observed every 50 or 60 units approximately. It is found in wood but it is also the main constituent of konjac gum. Konjac (*Amorphophallus konjac*) is a plant of the Araceae family.

Chemical structure of konjac gum:

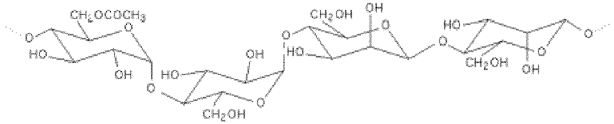

The products which can be used according to the invention are, for example, sold under the Propol® and Rheolex® names by Shimizu or, for example, under the name KG1220C by Kevin Food Ingredient.

According to the invention, the polysaccharide(s) are preferably chosen from glucomannans and xanthans.

The polysaccharide(s) according to the invention are preferably present in the composition in an amount ranging from 0.01% to 20% by weight, preferably from 0.05% to 15% by weight and more preferably from 0.5% to 10% by weight, with respect to the total weight of the composition.

### Anionic fixing polymers

The composition according to the invention also comprises at least one anionic fixing polymer.

Within the meaning of the present invention, the term "fixing polymer" is thus understood to mean a polymer capable of conferring a shape to a head of hair and/or of retaining the acquired shape of the head of hair.

The anionic fixing polymers generally used are polymers comprising groups derived from carboxylic acid, sulfonic acid or phosphoric acid and have a number-average molecular weight of between approximately 500 and 5 000 000.

The anionic fixing polymers having carboxylic groups which are preferred according to the invention are:
A) copolymers of acrylic acid and of acrylamide sold in the form of their sodium salts under the names Reten 421, 423 or 425 by Hercules or the sodium salts of polyhydroxycarboxylic acids;
B) copolymers of acrylic or methacrylic acid with a monoethylenic monomer, such as ethylene, styrene, vinyl esters, acrylic or methacrylic acid esters, optionally grafted onto a polyalkylene glycol, such as polyethylene glycol, and optionally crosslinked. Such polymers are described in particular in French Patent 1 222 944 and German Application 2 330 956, the copolymers of this type comprising an optionally N-alkylated and/or N-hydroxyalkylated acrylamide unit in their chain, as are described in particular in Luxembourgian Patent Applications 75370 and 75371, or provided under the Quadramer name by American Cyanamid. Mention may also be made of acrylic acid/ethyl acrylate/N-*tert*-butylacrylamide terpolymers, such as Ultrahold Strong, sold by BASF. Mention may also be made of copolymers of acrylic acid and of C₁-C₄ alkyl methacrylate and terpolymers of vinylpyrrolidone, of acrylic acid and of C₁-C₂₀ alkyl methacrylate, for example lauryl methacrylate, such as the product sold by ISP under the name Acrylidone® LM, and methacrylic acid/ethyl acrylate/tert-butyl acrylate terpolymers, such as the product sold under the name Luvimer® 100 P by BASF.
   Mention may also be made of the methacrylic acid/acrylic acid/ethyl acrylate/methyl methacrylate copolymers in aqueous dispersion sold under the name Amerhold® DR 25 by Amerchol;
C) crotonic acid copolymers, such as those comprising, in their chain, vinyl acetate or propionate units and optionally other monomers, such as allyl or methallyl esters, vinyl ether or vinyl ester of a linear or branched saturated carboxylic acid comprising a long hydrocarbon chain, such as those comprising at least 5 carbon atoms, it optionally being possible for these polymers to be grafted or crosslinked, or also another monomer which is a vinyl, allyl or methallyl ester of an α- or β-cyclic carboxylic acid. Such polymers are described, inter alia, in French Patents 1 222 944, 1 580 545, 2 265 782, 2 265 781, 1 564 110 and 2 439 798. Commercial products coming within this category are the Resins 28-29-30, 26-13-14 and 28-13-10 sold by Akzo Nobel;
D) copolymers of monounsaturated C₄-C₈ carboxylic acids or anhydrides chosen from:
   - copolymers comprising (i) one or more maleic, fumaric or itaconic acids or anhydrides and (ii) at least one monomer chosen from vinyl esters, vinyl ethers, vinyl halides, phenylvinyl derivatives, or acrylic acid and its esters, the anhydride functional groups of these copolymers optionally being monoesterified or monoamidated. Such polymers are described in particular in US Patents 2 047 398, 2 723 248 and 2 102 113, and GB Patent 839 805. Commercial products are in particular those sold under the names Gantrez® AN or ES by ISP;
   - copolymers comprising (i) one or more maleic, citraconic or itaconic anhydride units and (ii) one or more monomers chosen from allyl or methallyl esters, optionally comprising one or more acrylamide, methacrylamide, α-olefin, acrylic or methacrylic ester, acrylic or methacrylic acid or vinylpyrrolidone groups in their chain,
   the anhydride functional groups of these copolymers optionally being monoesterified or monoamidated.
   These polymers are, for example, described in French Patents 2 350 384 and 2 357 241 of the Applicant Company;
E) polyacrylamides comprising carboxylate groups;
F) homopolymers and copolymers comprising sulfonic groups, such as polymers comprising vinylsulfonic, styrenesulfonic, naphthalenesulfonic or acrylamidoalkylsulfonic units. These polymers can in particular be chosen from:
   - salts of polyvinylsulfonic acid having a molecular weight of between approximately 1000 and 100 000, as well as copolymers with an unsaturated comonomer, such as acrylic or methacrylic acids and their esters, as well as acrylamide or its derivatives, vinyl ethers and vinylpyrrolidone;
   - salts of polystyrenesulfonic acid, such as the sodium salts sold, for example, under the names Flexan® 500 and Flexan® 130 by National Starch. These compounds are described in Patent FR 2 198 719;
   - salts of polyacrylamidesulfonic acids, such as those mentioned in Patent US 4 128 631 and more particularly the polyacrylamidoethylpropanesulfonic acid sold under the name Cosmedia Polymer HSP 1180 by Henkel.

Mention may be made, as other anionic fixing polymer which can be used according to the invention, of the branched block anionic polymer sold under the name Fixate G-100 L by Lubrizol.

According to the invention, the anionic fixing polymers are preferably chosen from copolymers of acrylic acid or acrylic esters, such as the acrylic acid/ethyl acrylate/N-tert-butylacrylamide terpolymers sold in particular under the name Ultrahold® Strong by BASF, copolymers derived from crotonic acid, such as the vinyl acetate/vinyl tert-butylbenzoate/crotonic acid terpolymers and the crotonic acid/vinyl acetate/vinyl neododecanoate terpolymers sold in particular under the name Resin 28-29-30 by National Starch, polymers derived from maleic, fumaric or itaconic acids or anhydrides with vinyl esters, vinyl ethers, vinyl halides, phenylvinyl derivatives, or acrylic acid and its esters, such as the methyl vinyl ether/monoesterified maleic anhydride copolymers sold, for example, under the name Gantrez® by ISP, the copolymers of methacrylic acid and of methyl methacrylate sold under the name Eudragit® L by Rohm Pharma, the copolymers of methacrylic acid and of ethyl acrylate sold under the name Luvimer® MAEX or MAE by BASF, the vinyl acetate/crotonic acid copolymers sold under the name Luviset CA 66 by BASF and the vinyl acetate/crotonic acid copolymers grafted by polyethylene glycol sold under the name Aristoflex® A by BASF, and the polymer sold under the name Fixate G-100 L by Lubrizol.

According to the invention, the anionic fixing polymer(s) are more preferably still chosen from crotonic acid copolymers.

Preferably, the anionic fixing polymer(s) are chosen from crotonic acid copolymers comprising, in their chain, vinyl acetate or propionate units and optionally other monomers, such as allyl or methallyl esters, vinyl ether or vinyl ester of a linear or branched saturated carboxylic acid comprising a long hydrocarbon chain.

Most preferably, the anionic fixing polymer is a vinyl acetate/crotonic acid/vinyl neodecanoate terpolymer.

The anionic fixing polymer(s) according to the invention are preferably present in the composition in an amount ranging from 0.1% to 25% by weight, preferably from 1% to 20% by weight and more preferably from 1.5% to 15% by weight, with respect to the total weight of the composition.

The composition according to the invention also comprises one or more liquid monoalcohols or polyols.

The term "liquid" is understood to mean a compound which is liquid at 25°C and at atmospheric pressure (760 mmHg or 1.013 x 10⁵ Pa).

Mention will in particular be made, as liquid monoalcohols, of C₁₋₄ monoalcohol(s), such as ethanol, isopropanol, tert-butanol or n-butanol.

The polyols which can be used in the composition according to the invention are compounds composed of a hydrocarbon chain optionally interrupted by one or more oxygen atoms and carrying at least two free hydroxyl groups, and having a molecular weight of less than 1000.

The hydrocarbon chain can be linear or branched and saturated or unsaturated.

Preferably, the hydrocarbon chain comprises from 3 to 50 carbon atoms and better still from 3 to 20 carbon atoms.

In an alternative form of the invention, the hydrocarbon chain is saturated.

Very particularly, the polyols of the invention comprise 2 or 3 free hydroxyl functional groups.

The free hydroxyl functional groups can be carried by contiguous carbon atoms or the carbon atoms carrying them can be separated by one or more other carbon atoms.

Use is preferably made, among these polyols, of 1,2-propanediol (or propylene glycol), 1,3-propanediol, glycerol, 3-(2-ethylhexyl)oxy-1,2-propanediol (or ethylhexylglycerin), 3-methyl-1,3,5-pentanetriol, 1,2,4-butanetriol, 1,5-pentanediol, 2-methyl-1,3-propanediol, 1,3-butanediol, 3-methyl-1,5-pentanediol, neopentyl glycol (2,2-dimethyl-1,3-propanediol), isoprene glycol (3-methyl-1,3-butanediol) and hexylene glycol (2-methyl-2,4-pentanediol), diethylene glycol, dipropylene glycol, tripropylene glycol or polyethylene glycols of formula H(OCH₂CH₂)ₙOH with n ranging from 4 to 16.

Preferably, the liquid monoalcohol or polyol is ethanol.

The content of liquid monoalcohols or polyols is greater than or equal to 20% by weight, with respect to the total weight of the composition, and preferably ranges from 20% to 95% by weight, more preferably from 30% to 90% by weight and better still from 50% to 80% by weight, with respect to the total weight of the composition.

The composition according to the invention can also comprise a hair-conditioning agent.

In the context of the present patent application, the term "hair-conditioning agent" is understood to mean any agent having the function of improving the cosmetic properties of the hair, in particular the softness, sheen, disentangling, feel, smoothness or static electricity.

The conditioning agents can be provided in the liquid, semi-solid or solid form, such as, for example, oils, waxes or gums.

The conditioning agents can be chosen from fatty substances, such as C₆-C₁₆ hydrocarbons or hydrocarbons having more than 16 carbon atoms and in particular alkanes, oils of animal origin, oils of vegetable origin, glycerides or oils which are fluorinated and of synthetic origin, fatty alcohols, fatty acid and/or fatty alcohol esters, non-silicone waxes, silicones, fatty amines, fatty acids, cationic polymers, amphoteric polymers, cationic proteins, cationic protein hydrolysates, compounds of ceramide type, cationic surfactants, and also mixtures of these various compounds.

The composition according to the invention can also additionally comprise one or more thickening agents other than the polysaccharide(s) having at least one mannose unit.

Within the meaning of the present invention, the term "thickening agent" is understood to mean an agent which, by its presence in the composition, makes it possible to increase the viscosity of the said composition by at least 10 cPs and preferably by at least 200 cPs, at 25°C and at a shear rate of 1 s⁻¹. This viscosity can be measured using a cone/plate viscometer (Haake R600 rheometer or the like).

The thickening agent(s) can be chosen from fatty acid amides obtained from C₁₀-C₃₀ carboxylic acid (monoisopropanolamide, diethanolamide or monoethanolamide of coconut acids, monoethanolamide of oxyethylenated alkyl ether carboxylic acid), polymeric thickeners and in particular non-ionic cellulose polymers (hydroxyethylcellulose, hydroxypropylcellulose, carboxymethylcellulose), gum arabic, gums of microbial origin (scleroglucan gum), crosslinked or non-crosslinked homopolymers and copolymers based on acrylic acid, methacrylic acid or acrylamidopropanesulfonic acid, associative polymers as described below, and their mixtures.

The associative polymer(s) which can be used according to the invention are water-soluble polymers which are capable, in an aqueous medium, of reversibly associating with one another or with other molecules.

Their chemical structure comprises hydrophilic regions and hydrophobic regions characterized by at least one fatty chain preferably comprising from 10 to 30 carbon atoms.

The associative polymer(s) which can be used according to the invention can be of anionic, cationic, amphoteric or non-ionic type, such as the polymers sold under the names Pemulen TR1 or TR2 by Goodrich (INCI: Acrylates/C10-30 Alkyl Acrylate Crosspolymer), Salcare SC90 by Ciba, Aculyn 22, 28, 33, 44 or 46 by Röhm & Haas, and Elfacos T210 and T212 by Akzo.

Among all the thickening agents mentioned, the additional thickening agent(s) are preferably chosen from polymers and better still from non-ionic cellulose polymers, in particular hydroxypropylcellulose and gum arabic.

The additional thickening agents can be present in the composition of the invention in an amount varying from 0.01% to 20% by weight and better still from 0.1% to 10% by weight, with respect to the total weight of the composition.

The composition can be aqueous or anhydrous.

For the purposes of the invention, a composition is anhydrous when it has a water content of less than 2% by weight and preferably less than 1% by weight relative to the total weight of the composition.

Preferably, an anhydrous composition contains no added water during the preparation of the composition, the only water present corresponding to the residual water provided by the ingredients mixed. Even more preferably, the anhydrous composition is free of water.

When it is aqueous, it preferably comprises from 5% to 70% by weight of water, more preferably still from 10% to 60% by weight of water and better still from 10% to 50% by weight of water, with respect to the total weight of the composition.

When it is aqueous, the pH of the composition used according to the invention generally varies from 2 to 6.5, preferably from 3 to 6.5 and more preferably still from 4 to 6.

Preferably, the composition according to the invention can comprise active agents conventionally employed in non-long-lasting hair shaping other than those described above and chosen from non-ionic surfactants, anionic surfactants, amphoteric surfactants, cationic polymers, direct dyes, in particular cationic or natural direct dyes, or oxidation dyes, organic or inorganic pigments, UV-screening agents, fillers, in particular pearlescent agents, TiO₂, clays, fragrances, peptizing agents, vitamins, amino acids, preservatives, pH agents, agents for long-lasting hair shaping, in particular thiol-based organic reducing agents, non-thiol-based organic reducing agents, and the like.

Of course, a person skilled in the art will take care to choose the optional additional compounds and/or their amounts in such a way that the advantageous properties of the compositions used according to the invention are not, or not substantially, detrimentally affected by the envisaged addition.

Preferably, the compositions are provided in the form of gels. Preferably, the compositions have a viscosity of greater than or equal to 4 Pa.s and better still ranging from 4 Pa.s to 500 Pa.s at a temperature of 25°C and at a shear rate of 1 s⁻¹ (measurable, for example, with a Haake RS600 rheometer).

The composition according to the invention can in particular be used in a rinse-out or leave-in application on the hair. Preferably, the composition is used in a rinse-out application on the hair.

Another subject-matter of the invention is a method for the treatment of keratinous fibres, such as the hair, comprising the application of the cosmetic composition according to the invention which has just been described on the keratinous fibres.

Preferably, the composition is applied to wet hair, for example immediately after the rinsing out of a shampoo and/or of a conditioner. The method according to the invention additionally comprises a stage of rinsing the hair after the application of the composition.

A stage of shaping the hair, in particular by hand, can be carried out after the rinsing stage.

The hair can then optionally be dried, in particular using a hairdryer. Alternatively, it can be dried in the open air.

The composition according to the invention can thus be applied directly under the shower and rinsed out using the water of the shower, with running water. The shaping can thus take place after rinsing out the composition, which avoids an additional stage of rinsing the hands after leaving the shower.

The invention is illustrated in more detail in the following examples, which are presented by way of illustration and without limitation of the invention.

### EXAMPLES

The following styling gel formulations according to the invention were prepared. The concentrations are expressed as percentages by weight of active materials in the final composition.

| | C1 | C2 | C3 | C4 | C5 | C6 |
|---|---|---|---|---|---|---|
| Vinyl acetate/crotonic acid/vinyl neodecanoate terpolymer ⁽¹⁾ | 5 | 10 | 7.5 | 7.5 | 7.5 | 7.5 |
| Glucomannan ⁽²⁾ | 2 | 2 | 2 | 4 | - | - |
| Ceratonia siliqua gum and sucrose ⁽³⁾ | - | - | - | - | 2 | 4 |
| Ethanol | 93 | 88 | 90.5 | 88.5 | 90.5 | 88.5 |

| | | | | | | |
|---|---|---|---|---|---|---|
| ⁽¹⁾Resyn 28-2930, sold by Akzo Nobel ⁽²⁾ KG1220C, sold by Kevin Food Ingredient ⁽³⁾ VISCOGUM BCR 12/250 sold by CARGILL. | | | | | | |

| | C7 | C8 | C9 | C10 | C11 | C12 |
|---|---|---|---|---|---|---|
| Vinyl acetate/crotonic acid/vinyl neodecanoate terpolymer ⁽¹⁾ | 7.5 | 7.5 | 10 | 7.5 | 7.5 | 7.5 |
| Glucomannan ⁽²⁾ | 2 | 1 | 2 | | | |
| Hydroxypropylcellulose ⁽³⁾ | 2 | 2 | 2 | 2 | 2 | 2 |
| Xanthan gum ⁽⁴⁾ | | 1 | 3 | 1 | 3 | 1 |
| Gum arabic ⁽⁵⁾ | | | | 1 | | 1 |
| Water | 20 | 20 | 10 | 20 | 20 | 30 |
| Ethanol | 68.5 | 68.5 | 73 | 68.5 | 67.5 | 68.5 |

| | | | | | | |
|---|---|---|---|---|---|---|
| ⁽¹⁾ Resyn 28-2930, sold by Akzo Nobel ⁽²⁾ KG1220C, sold by Kevin Food Ingredient ⁽³⁾ Klucel MF Pharm, sold by Ashland ⁽⁴⁾ Rhodicare XC, sold by Rhodia ⁽⁵⁾ Atomized Gum Arabic 396A | | | | | | |

Compositions C1 to C12 were evaluated with respect to water.

A cleaning shampooing is carried out on a cluster of hair with a length of 1.5 cm attached to a support, the arrangement representing a quarter of a head of hair. The hair is rinsed with running water. Approximately 1 g of one of compositions C1 to C12 is applied to half of the cluster of wet hair. Rinsing is again carried out and then drying is allowed to take place.

In comparison with the half of the lock devoid of product, the fixing obtained is immediate and results from the rinsing. Before the rinsing stage, the hair remains supple and malleable. During the rinsing stage, precipitation on the hair and immediate fixing occur. This is accentuated by the drying.

## Claims

1. Cosmetic composition, in particular a hair cosmetic composition, comprising one or more polysaccharide(s) having at least one mannose unit, one or more anionic fixing polymer(s) and one or more liquid monoalcohols or polyols, the content of liquid monoalcohols or polyols being greater than or equal to 20% by weight of the total weight of the composition, the pH of the composition, when it is aqueous, being less than or equal to 6.5.

2. Cosmetic composition according to Claim 1, in which the polysaccharide(s) having at least one mannose unit are chosen from xanthans, galactomannans, glucomannans and their derivatives.

3. Cosmetic composition according to Claim 1 or Claim 2, in which the polysaccharide(s) are galactomannans chosen from guar, tara or locust bean gums.

4. Cosmetic composition according to Claim 1 or Claim 2, in which the polysaccharide(s) are glucomannans chosen from konjac gums.

5. Composition according to any one of the preceding claims, in which the content of polysaccharides having at least one mannose unit ranges from 0.01% to 20% by weight, preferably from 0.05% to 15% by weight and more preferably from 0.5% to 10% by weight, with respect to the total weight of the composition.

6. Composition according to any one of the preceding claims, in which the anionic fixing polymer(s) are chosen from copolymers of acrylic and methacrylic acid or their salts, crotonic acid copolymers, copolymers of monounsaturated C₄-C₈ carboxylic acids or anhydrides, polyacrylamides comprising carboxylate groups, homopolymers or copolymers comprising sulfonic groups, anionic polyurethanes, and anionic grafted silicone polymers.

7. Composition according to any one of the preceding claims, in which the anionic fixing polymer(s) are chosen from crotonic acid copolymers, and more preferably from crotonic acid copolymers comprising, in their chain, vinyl acetate or propionate units and optionally other monomers, such as allyl or methallyl esters, vinyl ether or vinyl ester of a linear or branched saturated carboxylic acid comprising a long hydrocarbon chain, and even more preferably is a vinyl acetate/crotonic acid/vinyl neodecanoate terpolymer.

8. Composition according to any one of the preceding claims, in which the content of anionic fixing polymer(s) ranges from 0.1% to 25% by weight, preferably from 1% to 20% by weight and more preferably from 1.5% to 15% by weight, with respect to the total weight of the composition.

9. Composition according to any one of the preceding claims, in which the liquid monoalcohol(s) or polyol(s) are chosen from ethanol, isopropanol, propylene glycol, butylene glycol, glycerol, polyol ethers, and their mixtures, the liquid monoalcohol or polyol preferably being ethanol.

10. Composition according to any one of the preceding claims, in which the content of liquid monoalcohol or polyol ranges from 20% to 95% by weight, more preferably from 30% to 90% by weight and better still from 50% to 80% by weight, with respect to the total weight of the composition.

11. Composition according to any one of the preceding claims, comprising one or more thickening agents other than the polysaccharide(s) having at least one mannose unit preferably chosen from polymers, better still from non-ionic cellulose polymers, in particular hydroxypropylcellulose and gum arabic.

12. Composition according to the preceding claim, in which the additional thickening agents are present in an amount varying from 0.01% to 20% by weight and better still from 0.1 % to 10% by weight, with respect to the total weight of the composition.

13. Composition according to any one of the preceding claims, **characterized in that** it is aqueous and preferably comprises from 5% to 70% by weight of water, more preferably still from 10% to 60% by weight of water and better still from 10% to 50% by weight of water, with respect to the total weight of the composition.

14. Composition according to any one of Claims 1 to 12, **characterized in that** it is anhydrous.

15. Composition according to any one of Claims 1 to 13, which exhibits a pH ranging from 2 to 6.5, preferably from 3 to 6.5 and more preferably still from 4 to 6.

16. Method for shaping the hairstyle, in which a composition according to any one of the preceding claims is applied to hair, the method further comprises a stage of rinsing the hair after the application of the composition, optionally followed by shaping the hair, also optionally followed by drying.

17. Method according to the preceding claim, in which a composition according to any one of Claims 1 to 15 is applied under the shower.

## Patentansprüche

1. Kosmetische Zusammensetzung, insbesondere haarkosmetische Zusammensetzung, umfassend ein oder mehrere Polysaccharide mit mindestens einer Mannose-Einheit, ein oder mehrere anionische fixierende Polymere und einen oder mehrere flüssige Monoalkohole oder ein oder mehrere flüssige Polyole, wobei der Gehalt an flüssigen Monoalkoholen oder Polyolen größer oder gleich 20 Gew.-% des Gesamtgewichts der Zusammensetzung ist, wobei der pH-Wert der Zusammensetzung, wenn sie wässrig ist, kleiner oder gleich 6,5 ist.

2. Kosmetische Zusammensetzung nach Anspruch 1, wobei das Polysaccharid bzw. die Polysaccharide mit mindestens einer Mannose-Einheit aus Xanthanen, Galactomannanen, Glucomanneann und Derivaten davon ausgewählt ist bzw. sind.

3. Kosmetische Zusammensetzung nach Anspruch 1 oder Anspruch 2, wobei es sich bei dem Polysaccharid bzw. den Polysacchariden um Galactomannane, die aus Guar-, Tara- und Johannisbrotgummen ausgewählt sind, handelt.

4. Kosmetische Zusammensetzung nach Anspruch 1 oder Anspruch 2, wobei es sich bei dem Polysaccharid bzw. den Polysacchariden um Glucomannane, die aus Konjak-Gummen ausgewählt sind, handelt.

5. Zusammensetzung nach einem der vorhergehenden Ansprüche, wobei der Gehalt an Polysaccharid mit mindestens einer Mannose-Einheit im Bereich von 0,01 bis 20 Gew.-%, vorzugsweise von 0,05 bis 15 Gew.-% und weiter bevorzugt von 0,5 bis 10 Gew.-%, bezogen auf das Gesamtgewicht der Zusammensetzung, liegt.

6. Zusammensetzung nach einem der vorhergehenden Ansprüche, wobei das anionische fixierende Polymer bzw. die anionischen fixierenden Polymere aus Copolymeren von Acryl- und Methacrylsäure oder Salzen davon, Crotonsäure-Copolymeren, Copolymeren von einfach ungesättigten C₄-C₈-Carbonsäuren und -Carbonsäureanhydriden, Polyacrylamiden mit Carboxylatgruppen, Homopolymeren oder Copolymeren mit Sulfonsäuregruppen, anionischen Polyurethanen und anionischen Silikonpfropfpolymeren ausgewählt ist bzw. sind.

7. Zusammensetzung nach einem der vorhergehenden Ansprüche, wobei das anionische fixierende Polymer bzw. die anionischen fixierenden Polymere aus Crotonsäure-Copolymeren und weiter bevorzugt aus Crotonsäure-Copolymeren, die in ihrer Kette Vinylacetat- oder Vinylpropionat-Einheiten und gegebenenfalls andere Monomere, wie Allyl- oder Methallylester, Vinylether oder Vinylester einer linearen oder verzweigten gesättigten Carbonsäure mit einer langen Kohlenwasserstoffkette umfassen, ausgewählt ist bzw. sind und noch weiter bevorzugt ein Vinylacetat/Crotonsäure/Vinylneodecanoat-Terpolymer ist.

8. Zusammensetzung nach einem der vorhergehenden Ansprüche, wobei der Gehalt an anionischem fixierendem Polymer bzw. anionischen fixierenden Polymeren im Bereich von 0,1 bis 25 Gew.-%, vorzugsweise von 1 bis 20 Gew.-% und noch weiter bevorzugt von 1,5 bis 15 Gew.-%, bezogen auf das Gesamtgewicht der Zusammensetzung, liegt.

9. Zusammensetzung nach einem der vorhergehenden Ansprüche, wobei der flüssige Monoalkohol bzw. die flüssigen Monoalkohole oder das flüssige Polyol bzw. die flüssigen Polyole aus Ethanol, Isopropanol, Propylenglykol, Butylenglykol, Glycerin, Polyolethern und Mischungen davon ausgewählt ist bzw. sind, wobei es sich bei dem flüssigen Monoalkohol oder Polyol vorzugsweise um Ethanol handelt.

10. Zusammensetzung nach einem der vorhergehenden Ansprüche, wobei der Gehalt an flüssigem Monoalkohol oder Polyol im Bereich von 20 bis 95 Gew.-%, weiter bevorzugt von 30 bis 90 Gew.-% und noch besser von 50 bis 80 Gew.-%, bezogen auf das Gesamtgewicht der Zusammensetzung, liegt.

11. Zusammensetzung nach einem der vorhergehenden Ansprüche, umfassend ein oder mehrere Verdickungsmittel, die von dem Polysaccharid bzw. den Polysacchariden mit mindestens einer Mannose-Einheit verschieden sind, vorzugsweise ausgewählt aus Polymeren, noch besser aus nicht ionischen Cellulosepolymeren, insbesondere Hydroxypropylcellulose und Gummi arabicum.

12. Zusammensetzung nach dem vorhergehenden Anspruch, wobei die zusätzlichen Verdickungsmittel in einer Menge im Bereich von 0,01 bis 20 Gew.-% und noch besser von 0,1 bis 10 Gew.-%, bezogen auf das Gesamtgewicht der Zusammensetzung, vorliegen.

13. Zusammensetzung nach einem der vorhergehenden Ansprüche, **dadurch gekennzeichnet, dass** sie wässrig ist und vorzugsweise 5 bis 70 Gew.-% Wasser, noch weiter bevorzugt 10 bis 60 Gew.-% Wasser und noch besser 10 bis 50 Gew.-% Wasser, bezogen auf das Gesamtgewicht der Zusammensetzung, umfasst.

14. Zusammensetzung nach einem der Ansprüche 1 bis 12, **dadurch gekennzeichnet, dass** sie wasserfrei ist.

15. Zusammensetzung nach einem der Ansprüche 1 bis 13, die einen pH-Wert im Bereich von 2 bis 6,5, vorzugsweise von 3 bis 6,5 und noch weiter bevorzugt von 4 bis 6 aufweist.

16. Verfahren zur Gestaltung der Frisur, bei dem man eine Zusammensetzung nach einem der vorhergehenden Ansprüche auf Haar aufbringt, wobei das Verfahren ferner eine Stufe des Spülens des Haars nach dem Aufbringen der Zusammensetzung umfasst, gegebenenfalls mit anschließender Gestaltung des Haars sowie gegebenenfalls mit anschließender Trocknung.

17. Verfahren nach dem vorhergehenden Anspruch, bei dem man eine Zusammensetzung nach einem der Ansprüche 1 bis 15 unter der Dusche aufbringt.

## Revendications

1. Composition cosmétique, notamment capillaire, comprenant un ou plusieurs polysaccharides ayant au moins un motif mannose, un ou plusieurs polymères fixants anioniques et un ou plusieurs monoalcools ou polyols liquides, la teneur en monoalcools ou polyols liquides étant supérieure ou égale à 20% en poids du poids total de la composition, le pH de la composition, lorsqu'elle est aqueuse, étant inférieur ou égal à 6,5.

2. Composition cosmétique selon la revendication 1, dans laquelle le ou les polysaccharides ayant au moins un motif mannose sont choisis parmi les xanthanes, les galactomannanes, les glucomannanes, et leurs dérivés.

3. Composition cosmétique selon les revendications 1 ou 2, dans laquelle le ou les polysaccharides sont des galactomannanes choisis parmi les gommes de guar, de tara ou de caroube.

4. Composition cosmétique selon les revendications 1 ou 2, dans laquelle le ou les polysaccharides sont des glucomannanes choisis parmi les gommes de konjac.

5. Composition selon l'une quelconque des revendications précédentes, dans laquelle la teneur en polysaccharides ayant au moins un motif mannose va de 0,01 à 20%, de préférence de 0,05 à 15%, de préférence encore de 0,5 à 10% en poids par rapport au poids total de la composition.

6. Composition selon l'une quelconque des revendications précédentes, dans laquelle le ou les polymères fixants anioniques sont choisis parmi les copolymères d'acide acrylique et méthacrylique ou leurs sels, les copolymères d'acide crotonique, les copolymères d'acides ou d'anhydrides carboxyliques monoinsaturés en C₄-C₈, les polyacrylamides à groupements carboxylates, les homopolymères ou copolymères à groupes sulfoniques, les polyuréthanes anioniques, et les polymères siliconés greffés anioniques.

7. Composition selon l'une quelconque des revendications précédentes, dans laquelle le ou les polymères fixants anioniques sont choisis parmi les copolymères d'acide crotonique, et plus préférablement parmi les copolymères d'acide crotonique comprenant, dans leur chaîne, des motifs d'acétate ou de propionate de vinyle et éventuellement d'autres monomères, tels que les esters d'allyle ou de méthallyle, un vinyléther ou un vinylester d'un acide carboxylique saturé linéaire ou ramifié comprenant une longue chaîne hydrocarbonée, et encore plus préférablement il s'agit d'un terpolymère d'acétate de vinyle/acide crotonique/néodécanoate de vinyle.

8. Composition selon l'une quelconque des revendications précédentes, dans laquelle la teneur en polymères fixants anioniques va de 0,1 à 25%, de préférence de 1 à 20%, de préférence encore de 1,5 à 15% en poids par rapport au poids total de la composition.

9. Composition selon l'une quelconque des revendications précédentes, dans laquelle le ou les monoalcools ou polyols liquides sont choisis parmi l'éthanol, l'isopropanol, le propylèneglycol, le butylène glycol, le glycérol , les éthers de polyols et leurs mélanges, de préférence le monoalcool ou polyol liquide étant l'éthanol.

10. Composition selon l'une quelconque des revendications précédentes, dans laquelle la teneur en monoalcool ou polyol liquide va de 20 à 95% en poids, de préférence encore de 30 à 90% en poids, mieux de 50 à 80% en poids par rapport au poids total de la composition.

11. Composition selon l'une quelconque des revendications précédentes, comprenant un ou plusieurs agents épaississants différents des polysaccharides ayant au moins un motif mannose choisis de préférence parmi les polymères, mieux parmi les polymères cellulosiques non ioniques, notamment l'hydroxypropylcellulose et la gomme arabique.

12. Composition selon la revendication précédente, dans laquelle les agents épaississants additionnels sont présents dans une quantité variant de 0,01 à 20% en poids, mieux de 0,1 à 10% en poids par rapport au poids total de la composition.

13. Composition selon l'une quelconque des revendications précédentes, **caractérisée en ce qu'**elle est aqueuse, et comprend de préférence de 5% à 70% d'eau en poids, encore plus préférentiellement de 10à 60% d'eau en poids, mieux de 10 à 50% d'eau en poids par rapport au poids total de la composition.

14. Composition selon l'une quelconque des revendications 1 à 12, **caractérisée en ce qu'**elle est anhydre.

15. Composition selon l'une quelconque des revendications 1 à 13, qui présente un pH allant de 2 à 6,5, de préférence de 3 à 6,5, et encore plus préférentiellement de 4 à 6.

16. Procédé de mise en forme de la coiffure, dans lequel on applique une composition selon l'une quelconque des revendications précédentes sur les cheveux, le procédé comprenant en outre une étape de rinçage des cheveux après l'application de la composition, puis éventuellement de mise en forme des cheveux, puis éventuellement encore de séchage.

17. Procédé selon la revendication précédente, dans lequel on applique une composition selon l'une quelconque des revendications 1 à 15 sous la douche.
